Europäisches Patentamt

European Patent Office

Office européen des brevets

(19)

(11) Veröffentlichungsnummer : **0 107 857**
**B1**

(12) **EUROPÄISCHE PATENTSCHRIFT**

(45) Veröffentlichungstag der Patentschrift :
30.12.86

(51) Int. Cl.⁴ : **C 07 D307/92//** C07C121/30, C07C57/03

(21) Anmeldenummer : 83110737.0

(22) Anmeldetag : 27.10.83

(54) Verfahren zur Herstellung von 8.12-Epoxy-13.14.15.16-tetranorlabdan.

(30) Priorität : 28.10.82 DE 3240054

(43) Veröffentlichungstag der Anmeldung :
09.05.84 Patentblatt 84/19

(45) Bekanntmachung des Hinweises auf die Patenterteilung : 30.12.86 Patentblatt 86/52

(84) Benannte Vertragsstaaten :
CH DE FR LI NL

(56) Entgegenhaltungen :
CHEMISCHE BERICHTE, Band 93, Nr. 11, 3. November 1960, Seiten 2663-2667, Weinheim, DE.
JOURNAL OF THE AMERICAN CHEMICAL SOCIETY, Band 77, Nr. 19, 5. Oktober 1955, Seiten 5068-5077, Columbus, Ohio, USA
CHEMICAL ABSTRACTS, Band 93, Nr. 11, 15. September 1980, Seite 756, Nr. 114751w, Columbus, Ohio, USA
CHEMICAL ABSTRACTS, Band 95, Nr. 23, 7. Dezember 1981, Seite 708, Nr. 204213t, Columbus, Ohio, USA
MONATSHEFTE FÜR CHEMIE, Band 88, 1957, Seiten 587-596, Wien, AT.

(73) Patentinhaber : **Consortium für elektrochemische Industrie GmbH**
**Zielstattstrasse 20**
**D-8000 München 70 (DE)**

(72) Erfinder : **Staiger, Gerhard, Dr. Dipl.-Chem.**
**Staudingerstrasse 65**
**D-8000 München 83 (DE)**
Erfinder : **Macri, Antonio**
**Kirchenstrasse 62**
**D-8000 München 80 (DE)**

## Beschreibung

Die Erfindung betrifft ein Verfahren zur Herstellung von 8.12-Epoxy-13.14.15.16-tetranorlabdan durch

a) Cyclisieren von Homofarnesylsäure zu Norambreinolid
b) Umsetzung von Norambreinolid mit Lithiumaluminiumhydrid zu 8-Hydroxy-13.14.15.16-tetranorlabdan-12-ol und
c) Cyclisieren des Diols zu 8.12-Epoxy-13.14.15.16-tetranorlabdan.

Es ist bekannt, daß die stereoisomeren Formen bzw. deren Gemische von 8.12-Epoxy-13.14.15.16-tetranorlabdan Ambra-Geruch aufweisen. Es sind auch bereits Versuche bekanntgeworden, diesen begehrten Ambra-Riechstoff synthetisch herzustellen. So berichten R.C. Cambie et al. in Austr. J. Chem. *24*, 582-591 (1971) von einem partialsynthetischen Weg, ausgehend von dem allerdings schwer zugänglichen Manoyloxid. Auch U.E. Sibierteseva et al., Zitatstelle Chem. Abs. 93 : 114751 w beschreiben die Synthese von 8.12-Epoxy-13.14.15.16-tetranorlabdan, wobei das weniger zugängliche Sclareol als Ausgangsverbindung eingesetzt wird. Norambreinolid wird zum 8-Hydroxy-13.14-15.16-tetranorlabdan reduziert und anschließend in Gegenwart von Toluolsulfonsäure cyclisiert. Unter diesen stark sauren Bedingungen entsteht jedoch in großen Mengen ein anderes Dehydratationsprodukt als das Zielprodukt. Das Cyclisieren von Homofarnesylsäure in Gegenwart von konzentrierter Ameisensäure und konzentrierter $H_2SO_4$ ergibt gemäß G. Lucius, Chem. Ber. *93*, 2663-2667 (1960) ein Lactongemisch, das kaum Norambreinolid enthält. A. Saito et al., Chem. Lett. *1981*, 757-760, cyclisiertenTrans-β-monocyclohomofarnesylsäure in Gegenwart von Zinntetrachlorid zu einem stereospezifischen Norambreinolid. Es war nicht zu erwarten, daß noch eine zweckmäßige Ausbeute erzielt wird, wenn im Hinblick auf das Zielprodukt nicht stereospezifische Ausgangsmaterialien eingesetzt werden. Dies wird auch durch Stork et al., JACS *77*, 5068-77 (1955) bestätigt, wo Farnesylessigsäure in Gegenwart von $SnCl_4$ zu Ambreinolid in einer Ausbeute unter 10 % cyclisiert wurde.

Aufgabe der Erfindung war es, einen Syntheseweg für 8.12-Epoxy-13.14.15.16-tetranorlabdan mit gesteigerter Ausbeute aufzuzeigen. Insbesondere war es Aufgabe der Erfindung, die stark ausbeutemindernden Syntheseschritte zu verbessern.

Es wurde nun gefunden, daß die Cyclisierung von Homofarnesylsäure zu Norambreinolid in Gegenwart von $SnCl_4$ erhebliche Ausbeuteverbesserungen erbringt. Weiterhin wurde gefunden, daß die Cyclisierung von 8-Hydroxy-13.14.15.16-tetranorlabdan-12-ol zum Zielprodukt in Gegenwart von $POCl_3$ in guter Ausbeute und insbesondere ohne Beeinträchtigung der olfaktorischen Qualität verläuft, die bei der bekannten Cyclisierung in Gegenwart von p-Toluolsulfonsäurechlorid in Kauf genommen werden muß.

Gegenstand der Erfindung ist ein Verfahren zur Herstellung von 8.12-Epoxy-13.14.15.16-tetranorlabdan durch

a) Cyclisieren von Homofarnesylsäure zu Norambreinolid
b) Umsetzung von Norambreinolid mit Lithiumaluminiumhydrid zu 8-Hydroxy-13.14.15.16-tetranorlabdan-12-ol und
c) Cyclisieren des Diols zu 8.12-Epoxy-13.14.15.16-tetranorlabdan,

das dadurch gekennzeichnet ist, daß Homofarnesylsäure in Gegenwart von $SnCl_4$ zu Norambreinolid und 8-Hydroxy-13.14.15.16-tetranorlabdan-12-ol in Gegenwart von $POCl_3$ zu 8.12-Epoxy-13.14.15.16-tetranorlabdan cyclisiert werden.

Eine besonders bevorzugte Ausführungsform der Erfindung umfaßt die folgenden Verfahrensschritte :

a) Umsetzen von Farnesylbromid zum Farnesylcyanid
b) Verseifen von Farnesylcyanid zur Homofarnesylsäure,
c) Cyclisieren von Homofarnesylsäure zum Norambreinolid in Gegenwart von $SnCl_4$,
d) Umsetzen von Norambreinolid mit Lithiumaluminiumhydrid zu 8-Hydroxy-13.14.15.16-tetranorlabdan-12-ol und
e) Cyclisieren von 8-Hydroxy-13.14.15.16-tetranorlabdan-12-ol in Gegenwart von $POCl_3$.

Das als Ausgangsverbindung eingesetzte Farnesylbromid ist in an sich bekannter Weise durch Umsetzen von Bromiden, z. B. Phosphortribromid mit Farnesol zugänglich. Farnesol ist eine handelsübliche Substanz. Als alternative Basischemikalie kann weiterhin Nerolidol eingesetzt werden, das durch Behandeln mit Phosphortribromid in Farnesylbromid übergeführt wird.

Homofarnesylcyanid (4.8.12-trimethyltrideca-3.7.11-triennitril) ist in an sich bekannter Weise durch Behandeln von Farnesylbromid mit Alkalimetallcyanidlösungen zugänglich. Es wird am besten bei 1,5- bis 3-fachem molarem Überschuß an Cyanid gearbeitet. Besonders hohe Ausbeuten werden in einem 2-phasigen Reaktionsgemisch durch phasentransferkatalysierte Reaktion erzielt. Als inerte organische Lösungsmittel kommen beispielsweise Halogenwasserstoffe wie Dichlormethan, Chlorbenzol oder Kohlenwasserstoffe wie Benzol, Toluol und Xylol in Betracht. Beispiele für Phasentransferkatalysatoren sind Tetrabutylammoniumbromid bzw. -jodid oder Benzyltrimethylammoniumchlorid. Die zweite Phase bilden konzentrierte, wäßrige Alkalimetallcyanidlösungen. Die Reaktionstemperaturen betragen im allgemeinen 0 bis 120 °C, bevorzugt 40 bis 90 °C.

Homofarnesylsäure (4.8.12.-trimethyltrideca-3.7.11-triensäure) ist in an sich bekannter Weise durch Verseifen von Homofarnesylcyanid zugänglich. Am besten wird alkalisch verseift, beispielsweise in Alkohol/Wasser/Alkalihydroxid-Lösungen bei Temperaturen von 20 bis 120 °C. Die gewünschte Homofarnesylsäure wird anschließend durch Ansäuern des alkalischen Reaktionsgemisches und Extrahieren der freien Säure aus dem Reaktionsgemisch gewonnen.

Die Cyclisierung von Homofarnesylsäure zum tricyclischen Lakton Norambreinolid erfolgt erfindungsgemäß in Gegenwart von SnCl₄. Die Cyclisierungsreaktion wird am besten in inerten organischen Lösungsmitteln, z. B. Halogenkohlenwasserstoffen, wie Dichlormethan oder Kohlenwasserstoffen, wie Cyclohexan bei Temperaturen von — 100 bis + 25 °C, insbesondere — 78 bis 0 °C durchgeführt. Die Menge an erfindungsgemäß eingesetztem SnCl₄ ist mindestens äquimolar, bezogen auf die eingesetzte Menge an Homofarnesylsäure. Vorzugsweise beträgt der Überschuß 1,5-2 Mol. Schließlich werden die anorganischen Bestandteile des Reaktionsgemisches durch Zugabe von Wasser abgetrennt und das gewünschte Norambreinolid aus der organischen Phase isoliert.

Die Umsetzung des Laktons Norambreinolid zu dem Diol 8-Hydroxy-13.14.15.16-tetranorlabdan-12-ol geschieht in an sich bekannter Weise mit Lithiumaluminiumhydrid in wasserfreier organischer Phase. Lithiumaluminiumhydrid wird, bezogen auf 1 Mol eingesetztes Norambreinolid in Mengen von 0,5 bis 2 Mol verwendet. Es werden Temperaturen von 0 bis 40 °C eingehalten. Zumeist wird eine Lösung von Lithiumaluminiumhydrid vorgelegt und Norambreinolid zudosiert. Geeignete Lösemittel sind Diethylether, Tetrahydrofuran, Dioxan, Ethylenglycoldimethylether. Schließlich wird die anorganische Phase mit Wasser und mäßig alkalisch wäßriger Lösung abgetrennt und das gewünschte Diol aus der organischen Phase isoliert.

Die Cyclisierung des Diols 8-Hydroxy-13.14.15.16-tetranorlabdan-12-ol geschieht erfindungsgemäß in Gegenwart von in Bezug auf eingesetztes Diol äquimolaren oder in etwa äquimolaren Mengen an POCl₃. Zweckmäßigerweise wird das Diol vorgelegt und Phosphorylchlorid zudosiert. Geeignete Lösungsmittel sind organische Basen, wie Pyridin, Piccolin, Lutidin.

Die Reaktionstemperaturen liegen im Bereich von — 10 bis 40 °C. Danach wird alkalisch gestellt und schließlich das Zielprodukt aus dem Reaktionsgemisch extrahiert.

Nach dem erfindungsgemäßen Verfahren gelingt es, 8.12-Epoxy-13.14.15.16-tetranorlabdan, ausgehend von handelsüblichen Substanzen, wie Farnesol oder Nerolidol, in wesentlich verbesserter Ausbeute zu gewinnen. Es wird ein Gemisch von drei diastereomeren Formen erhalten, das einen ausgeprägten Ambra-Geruch aufweist.

## Patentanspruch

Verfahren zur Herstellung von 8.12-Epoxy-13.14.15.16-tetranorlabdan durch

a) Cyclisieren von Homofarnesylsäure zu Norambreinolid

b) Umsetzung von Norambreinolid mit Lithiumaluminiumhydrid zu 8-Hydroxy-13.14.15.16-tetranorlabdan-12-ol und

c) Cyclisieren des Diols zu 8.12-Epoxy-13.14.15.16-tetranorlabdan,
dadurch gekennzeichnet, daß Homofarnesylsäure in Gegenwart von SnCl₄ zu Norambreinolid und 8-Hydroxy-13.14.15.16-tetranorlabdan-12-ol in Gegenwart von POCl₃ zu 8.12-Epoxy-13.14.15.16-tetranorlabdan cyclisiert werden.

## Claim

Process for the manufacture of 8,12-epoxy-13,14,15,16-tetranorlabdane by

a) cyclisation of homofarnesylic acid to form norambreinolide,

b) reaction of norambreinolide with lithium aluminium hydride to form 8-hydroxy-13,14,15,16-tetranorlabdan-12-ol, and

c) cyclisation of the diol to form 8,12-epoxy-13,14,15,16-tetranorlabdane,
characterised in that homofarnesylic acid is cyclised in the presence of SnCl₄ to form norambreinolide and 8-hydroxy-13,14,15,16-tetranorlabdan-12-ol is cyclised in the presence of POCl₃ to form 8,12-epoxy-13,14,15,16-tetranorlabdane.

## Revendication

Procédé de préparation du 8,12-époxy-13,14,15,16-tétranorlabdane par :

a) cyclisation de l'acide homofarnésylique en norambréinolide,

b) réaction du norambréinolide avec de l'hydrure de lithium et aluminium pour former le 8-hydroxy-13,14,15,16-tétranorlabdane-12-ol, et

c) cyclisation de ce diol en 8,12-époxy-13,14,15,16-tétranorlabdane,
procédé caractérisé en ce que l'on effectue la cyclisation de l'acide homofarnésylique en norambréinolide en présence de SnCl₄ et la cyclisation du 8-hydroxy-13,14,15,16-tétranorlabdane-12-ol en 8,12-époxy-13,14,15,16-tétranorlabdane en présence de POCl₃.